# EUROPÄISCHE PATENTSCHRIFT

(11) **EP 0 708 076 B1**
(45) Veröffentlichungstag und Bekanntmachung des Hinweises auf die Patenterteilung: **23.04.2003**
(21) Anmeldenummer: 95115901.1
(22) Anmeldetag: 09.10.1995
(51) Int. Cl.: C07C 205/06, C07C 201/08, C07C 205/12

(54) **Verfahren zur Dinitrierung von aromatischen Verbindungen**
Process for the dinitration of aromatic compounds
Procédé pour la dinitration de composés aromatiques

(30) Priorität: 17.10.1994 DE 4437047
(43) Veröffentlichungstag der Anmeldung: 24.04.1996
(73) Patentinhaber: BAYER AG, 51368 Leverkusen (DE)
(72) Erfinder: Pirkl, Hans-Georg, Dr., D-50679 Köln (DE); Schomäcker, Reinhard, Dr., D-51381 Leverkusen (DE); Klingler, Uwe, D-41539 Dormagen (DE); Schieb, Thomas, Dr., D-51503 Rösrath (DE); Wiechers, Gerhard, Dr., D-51381 Leverkusen (DE); Zimmermann, Jürgen, Dr., Walnut Creek, CA 94598 (US)

(56) Entgegenhaltungen:
- EP-A- 0 597 361
- US-A- 5 099 080

## Beschreibung

Die Erfindung betrifft ein Verfahren zur kontinuierlichen, direkten Herstellung von mehrfach nitrierten Aromaten durch Umsetzung von einem Aromaten mit einer Nitroniumionen-haltigen Lösung.

Die Herstellung von nitrierten Aromaten ist seit langem Gegenstand zahlreicher Veröffentlichungen und Patente. Bereits seit 1846 ist bekannt, daß aromatische Verbindungen durch eine Mischung aus Schwefel- und Salpetersäure, sogenannte Misch- oder Nitriersäure, zu den entsprechenden Aromaten umgesetzt werden können [¹].

Seit vielen Jahrzehnten werden beispielsweise Nitrobenzol, Dinitrobenzol, Nitrochlorbenzol, Nitrotoluol und Dinitrotoluol isotherm in Rührkesseln oder Schlaufenreaktoren mit einer Mischsäure aus Schwefelsäure und Salpetersäure technisch hergestellt. Nach einer Phasentrennung zwischen organischer und wäßriger Phase wird das nitrierte aromatische Produkt gewonnen und die Schwefelsäure durch Verdampfen von Wasser bei erhöhter Temperatur aufkonzentriert. Mehrfache Nitrierungen, beispielsweise Dinitrierungen, werden bis heute durch ein zweistufiges isothermes Nitrieren großtechnisch durchgeführt. [²], [³].

Die kontinuierliche (Mono-) Nitrierung von Aromaten wird bereits in [⁴] ausführlich dargestellt: Mischsäure oder getrennte Ströme von Schwefel- und Salpetersäure werden beispielsweise mit Benzol zusammen in einen Nitrierrührreaktor dosiert. Die zweiphasige Reaktionsmischung wird immerwährend gerührt und intensiv gekühlt, um die Reaktion möglichst isotherm zu führen. Aus dem Nitrierreaktor wird diese Mischung in einen weiteren, nachgeschalteten Rührreaktor gegeben oder direkt in einen Phasentrennapparat eingeleitet. Die organische Produktphase wird dort von der wäßrigen Schwefelsäurephase separiert und aufgearbeitet. Die durch das Salpetersäurewasser und Reaktionswasser verdünnte Schwefelsäure muß unter erheblichem Energieaufwand aufkonzentriert in den Reaktor zurückgeführt werden. Bei einer mehrfachen Nitrierung eines Aromaten wird dieser energetisch aufwendige Aufkonzentrierungsprozeß bei jeder einzelnen, nacheinander geschalteten, isothermen Mononitrierung durchgeführt. Gegebenenfalls wird, beispielsweise bei der Dinitrierung von Toluol, die Schwefelsäure von der Di- zur Mononitrierstufe geführt, so daß nur eine Schwefelsäure-Aufkonzentrierung benötigt wird.

Die physikalischen und chemischen Daten dieser technischen Nitrierbedingungen sowie die Modellvorstellungen zur Nitrierung mit Nitroniumionen-haltigen Lösungen sind in der Literatur im wesentlichen bekannt [⁵], [⁶], [⁷], [⁸]:

Die Nitrierung erfolgt in einer zweiphasigen Reaktion (organische Aromatenphase und wäßrige Nitriersäurephase) im wesentlichen in der wäßrigen Phase, so.daß die Löslichkeiten der Aromaten in der wäßrigen Phase, die Stofftransportgeschwindigkeit von Phase zu Phase und die intrinsische Reaktionsgeschwindigkeit insgesamt die global zu beobachtende Umsatzgeschwindigkeit beeinflussen. Somit liegt ein komplexes Reaktionssystem mit je nach Reaktionsführung kinetischer oder stofftransportlicher Geschwindigkeitskontrolle vor.

Für die aromatische Mononitrierung wurde von Albright et al. [⁹] ein Modell formuliert:
a) Nicht-nitrierter Aromat diffundiert aus der organischen Phase an die Phasengrenzfläche org./wäßrig
b) Nicht-nitrierter Aromat löst sich und diffundiert von der Phasengrenzfläche org./wäßrig in die wäßrige Phase
c) Salpetersäure diffundiert aus dem Kern der wäßrigen Phase Richtung Phasengrenzfläche
d) Während der Aromat in die wäßrige Phase eindringt und die HNO₃ ihm aus dem Kern der Flüssigphase entgegendiffundiert, reagiert der Aromat mit HNO₃ unter Bildung von Nitroaromat und Wasser
e) Gebildeter Nitroaromat diffundiert zurück durch die wäßrige Phase an die Phasengrenzfläche
f) Gebildeter Nitroaromat löst sich an der Phasengrenze in der organischen Phase und diffundiert von der Phasengrenzfläche in den Kern der organischen Phase
g) Gebildetes Wasser diffundiert vom Bildungsort in den Kern der wäßrigen Phase.

Da die konventionellen isothermen Monononitrierverfahren energetisch wenig optimiert sind, in dem die Reaktionswärme zunächst durch Kühlen abgeführt wird und die anschließende Säureaufkonzentrierung unter hoher Energiezufuhr gefahren wird, wurden schon früh Überlegungen zur adiabatischen Prozeßführung angestellt.

In US-A 2,256,999 (1941) wird eine adiabatische Mononitrierung einiger Aromaten, beispielsweise Benzol, als neues Nitrierverfahren vorgeschlagen. Wesentliche Merkmale dieses Prozesses sind, daß in einem oder mehreren Rührkesseln die zu nitrierende Verbindung überstöchiometrisch zugegeben wird, es somit zum vollständigen Verbrauch der Salpetersäure im Reaktor kommt. Anschließend wird die Schwefelsäure separiert, unter Nutzung der Reaktionswärme konzentriert und in den Reaktor rezykliert. Der verwendete Schwefelsäureanteil in der Mischsäure liegt bei 68 und 76 Gew.%.

US-A 4,021,498 (1977) konkretisiert ein solches Verfahren zur Mononitrierung und beschreibt, daß die adiabatische Mononitrierung mit einem Salpetersäureüberschuß bei Mischtemperaturen zwischen 40 und 80°C, Schwefelsäuregehalten von 60 bis 70 Gew.% und einer maximalen Temperatur von unter 145°C arbeitet. In US-A 4,091,042 (1978) werden speziell für die Mononitrierung von Benzol die Reaktorbetriebsbedingungen eingeschränkt. Es wird bei etwa 10 mol%igem Benzolüberschuß gegenüber HNO₃ und H₂SO₄-Gehalten von 58.5 bis 66,5 Gew.% gearbeitet.

In den Patenten US-A 2,256,999 (1941), US-A 4,021,498 (1977) und US-A 4,091,042 (1978) werden somit bereits adiabatische, kontinuierliche Prozesse zur Herstellung von Mononitroaromaten dargestellt.

Alternativ kann die Nitrierwärme gemäß US-A 3,928,475 (1975) und US-A 3,981,935 (1976) genutzt werden, in dem Benzoldampf und Salpetersäure in einen Schwefelsäure-haltigen Rührkessel dosiert werden und die verdampfenden Produkte Wasser und Nitrobenzol zusammen mit Benzol aus dem Reaktor gasförmig abgezogen werden. Beide Prozeßvarianten nutzen zwar die Reaktionswärme zur Wasserentfernung, verschenken jedoch einen Teil der Wärme, in dem sie große Mengen an Edukt mitverdampfen und kondensiert rezyklieren.

In EP-A 0,373,966 (1988; = US 284,700) wird die Mononitrierung von organischen Stoffen mit Mischsäure dargestellt, die zur Vermischung der beiden Phasen einen Tropfen erzeugenden Flüssigkeitsstrahl aus org. Stoff einsetzt. Das Nutzen eines Flüssigkeitsstrahls zur Flüssig/Flüssig-Vermischung bei der Mononitrierung ist bereits prinzipiell bekannt aus US-A 3,160,669 (1964). In EP-A 0,373,966 wird weiterhin mit einem HNO₃-Unterschuß gearbeitet, so daß der Aromat nicht vollständig im Reaktor nitriert wird. Im einzigen Beispiel wird in einem Reaktor mit Benzoleinspritzung über eine Düse lediglich ein Umsatz von 55,3% für HNO₃ und von 52,5% für Benzol erreicht.

In EP-A 0,436,443 (1990; = US-A 5,313,009(1994)) wird ein kontinuierlicher, adiabatischer Nitrierprozeß offenbart, der eine Mischsäure mit mindestens 55 mol% H₂SO₄ bei 0% HNO₃ und mindestens 82 mol% H₂SO₄ bei 18 mol% HNO₃ enthält. In Unteransprüchen wird eine Nitrierung mit einem Überschuß an Aromat gegenüber HNO₃ beansprucht. In der Beschreibung der Erfindung wird ausdrücklich betont, daß eine Dinitrierung vermieden werden sollte.

Alle bisher dargestellten Erfindungen zu adiabatischen Nitrierverfahren konzentrierten sich auf Benzol in ihren konkreten Beispielen und stellen Möglichkeiten zur Prozeßführung für eine Mononitrierung dar. Weitergehende Nitrierungen sind wesentlich schwieriger zu beherrschen, da hierzu erheblich drastischere Reaktionsund Schwefelsäureaufarbeitungsbedingungen benötigt werden, als sie bei einer Mononitrierung, beispielsweise von Benzol, auftreten [¹⁰]. Die zweite und insbesondere die dritte Nitrogruppe läßt sich schwerer in einen Aromaten einführen als die erste. So wählt man in der Praxis bis heute höhere Temperaturen und größere Säurekonzentrationen zur isothermen Herstellung von Di- gegenüber von Mononitroaromaten [¹¹].

In US-A 5,001,272 (1989) wird erstmals ein Verfahren zur Produktion eines dinitrierten Aromaten offenbart. Es gelingt, Toluol mit hochkonzentrierter wäßriger Salpetersäure ohne weitere Zusätze zu Dinitrotoluol umzusetzen. Es werden hohe molare Überschüsse an HNO₃ gegenüber Toluol von 5 bis 9 bei moderaten Temperaturen zwischen 40 und 70°C benötigt.

In US 4,918,250 (1989), US 5,057,632 (1990) und WO-A 92/06937 (1990) wird ein zweistufiger Prozeß zur Nitrierung von Toluol über die separierte Zwischenstufe Nitrotoluol zum Endprodukt Dinitrotoluol offenbart. Zur Dinitrierung wird mit einem hohen molaren Überschuß an hochkonzentrierter Salpetersäure gearbeitet.

In US-A 5,099,078 (1990), US-A 5,099,080 (1991) und US-A 5,245,092 (1992) wird ein Prozeß zur Dinitrierung von Toluol mit hochkonzentrierter Salpetersäure in einem einzigen Apparat offenbart. In US-A 5,099,080 (1991) wird dazu ein hoher molarer HNO₃-Überschuß eingestellt (HNO₃:Toluol = 12:1 bis 9:1) und bei Temperaturen von 0 bis 90°C nitriert. In US-A 5,245,092 (1992) wird der molare Überschuß an HNO₃ noch höher gewählt.

Erstmals wurde für eine Dinitrierung mit einer Mischsäure ein adiabatisches kontinuierliches Verfahren incl. der Nutzung der Reaktionswärme zur Verdampfung des Salpetersäure- und Produktwassers in DE-A 42 38 390 (1993) offenbart. Es wird eine Nitrierung mit einem Salpetersäure/Schwefelsäure-Gemisch mit einem H₂SO₄-Gewichtsanteil von 60 bis 90%, einem HNO₃-Gewichtsanteil von 5 bis 20% und einem Molverhältnis von HNO₃/Toluol von mindestens 2,0 beschrieben, wobei keine näheren Ausführungen zum zu verwendenden Reaktor gemacht werden. Es wird in Beispielen beschrieben, daß es in einem dünnen Rohrreaktor mit einem Innendurchmesser von 0,6 bzw. 0,99 mm und einer Länge von 20 m gelingt, Toluol zu Dinitrotoluol zu nitrieren. HNO₃ wird hierbei überstöchiometrisch bezüglich Toluol (Molverhältnis 2,15:1,0) zugesetzt und die Ausbeute beträgt mehr als 99% Dinitrotoluol (DNT). Mononitrotoluole und Trinitrotoluol treten nur in sehr kleinen Mengen (< 1%) auf. Der Reaktor wird in den Beispielen bei Durchsätzen von 1 bis 3 l/h mit sehr hohen Druckverlusten betrieben, die technisch schwer zu beherrschen sind. Das Scale Up eines solchen laminar durchströmten Reaktors ist nur durch eine technisch sehr aufwendige Vervielfältigung der einzelnen dünnen Rohrreaktoren möglich. Der Vorteil der adiabatischen Betriebsweise geht teilweise durch die Reaktorkonstruktion aufgrund der hohen Wärmeabfuhrverluste verloren und kann nur durch aufwendige Isoliermaßnahmen begrenzt werden.

Es bestand somit die Aufgabe, eine praktisch durchführbare Reaktionsführung zur adiabatischen Dinitrierung von Aromaten mit einer Mischsäure als Nitriermedium in einem einzigen Reaktorsystem zu entwickeln. Trotz der sehr unterschiedlichen Reaktionsbedingungen einer Mononitrierung und einer Dinitrierung einer aromatischen Verbindung sollen Reaktionsbedingungen eingestellt werden, die unter Berücksichtigung der Zersetzungsreaktionen der Produkte und der Minimierung der Energiekosten zu einer direkten Dinitrierung von Aromaten führt.

Bei heterogenen Reaktionssystemen wie beispielsweise der Nitrierung von Aromaten mit einer Mischsäure tritt häufig eine Hemmung der Reaktionsgeschwindigkeit durch den Stofftransport von einer in die andere Phase ein. Hierzu ist aus [¹²] bekannt, daß zwei nichtmischbare Flüssigkeiten mittels einer Druckdüse als Tröpfchen fein ineinander verteilt werden können. Durch die starke Vergrößerung der Grenzfläche zwischen den beiden Flüssigkeiten können chemische Reaktionen zwischen Reaktanden in verschiedenen Phasen umso schneller ablaufen, je homogener, d.h. feinerdisperser, sie verteilt werden. Bei hohen Energieeinträgen in ein zweiphasiges System, beispielsweise durch eine Düse, wird ein Flüssigkeitsstrahl unmittelbar nach der Düsenmündung in kleine Tropfen zerteilt. Die experimentellen Ergebnisse zeigen, daß über den Energieeintrag eines beliebigen Mischorganes und die Stoffdaten der beiden Flüssigkeiten die Tropfengrößen der dispersen Phase berechnet werden kann. Geeignete Mischorgane zur Dispergierung sind prinzipiell bekannt, beispielsweise kommen je nach den jeweiligen Dispergieranforderungen Strahlmischer, statische oder dynamische Mischer in Frage [¹³], [¹⁴].

Die Verminderung von unerwünschten Nebenprodukten bei komplexen Reaktionssystemen von mehreren Parallel- und Folgereaktionen durch die Mikromischung der Edukte, die schneller geschehen muß als die Edukte miteinander reagieren, ist seit langem in der Literatur bekannt [¹⁵], [¹⁶], [¹⁷]. Liegen die chemischen Reaktionsgeschwindigkeiten und die Vermischungsgeschwindigkeiten der Edukte in der gleichen Größenordnung, so kommt es zu einer komplexen Wechselwirkung zwischen den Kinetiken der Reaktionen und dem lokalen, von der Turbulenz bestimmten Vermischungsverhalten im Reaktor und am Mischorgan. Sind die Reaktionsgeschwindigkeiten sogar wesentlich schneller als die Mischgeschwindigkeiten, so werden die Ausbeuten deutlich durch die Vermischung, d.h. durch das lokale Geschwindigkeits- und Konzentrationsfeld der Reaktanden und somit von der Reaktorkonstruktion und Turbulenzstruktur, beeinflußt [¹⁸]. Geeignete Vorrichtungen zur schnellen Vermischung zweier Flüssigströme sind aus vielen Literaturangaben und Patenten bekannt [¹⁹]. Viele spezielle Vorrichtungen wurden für das flüssig/flüssig-Vermischen entwickelt. Einige Beispiele hierfür sind US-A 4 596 699; US-A 4 647 212; US-A 4 361 407 und EP-A 272 974. Außerdem wurden auch spezielle Vorrichtungen für die adiabatische Mononitrierung von Benzol in vermischenden Rohrreaktoren offenbart (EP-A 0,373,966, US-A 4,994,242 ).

Die in US-A 4,994,242 patentierte Vorrichtung stellt einen mit sphärischen Redispergierblechen aus Tantal ausgestatteten Rohrreaktor dar. Solche Redispergierelemente sind prinzipiell bekannt zur Führung von zweiphasigen Reaktionen, z.B. in Form von Siebblechen, Glockenböden, Düsenböden, Dispergatoren, Homogenisatoren, dyn. Mischorganen etc. [²⁰], [²¹], [²²]. Besondere Neuheit in US-A 4,994,242 stellt die hohe Stabilität der Redispergierelemente bei minimalem Materialeinsatz (Tantal) dar.

Es wurde jetzt gefunden, daß mehrfache Nitrierungen von Aromaten in einem einzigen Apparat unter adiabatischen Reaktionsbedingungen mit einer Mischsäure als Nitriermedium durchgeführt werden können, wobei prinzipiell alle Aromaten mehrfach bei Verweilzeiten unter 10 Minuten nitriert werden können. Gegenstand der Erfindung ist ein Verfahren zur kontinuierlichen, adiabatischen Herstellung von mehrfach nitrierten aromatischen Verbindungen in einem Mehrfachnitrierreaktor, dadurch gekennzeichnet, daß in einem einzigen Apparat eine aromatische Verbindung mit einem molaren Verhältnis von HNO₃ zu Aromat von 1,3 bis 3,5 di- oder höhernitriert wird, wobei die Erstdispergierung der Eduktströme in weniger als einer Sekunde zur Herstellung einer Emulsion als Reaktionsmedium durchgeführt wird, um bei der extrem schnellen Erstnitrierung die Stofftransporthemmung und die Bildung von Nebenprodukten (z.B. Nitrobenzoesäuren, Nitrokresole) zu minimieren, sowie durch wiederkehrendes Redispergieren der zur Koaleszenz neigenden Emulsion eine hohe spezifische Phasengrenzfläche aufrechterhalten wird, um eine hohe Raum-Zeit-Ausbeute zu erzielen. Nach diesem Verfahren können auch trinitrierte Aromaten hergestellt werden, die aber nicht Gegenstand der vorliegenden Anmeldung sind.

Die Nitroniumionen-haltige Lösung kann erfindungsgemäß auch in Teilmengen in den Reaktor geführt werden. Bevorzugte Zuführstellen für diese Teilmengenströme sind neben der Erstdispergierdüse die Redispergatoren der einzelnen Reaktorstufen entlang der Reaktorachse. Die Nitroniumionen-haltigen Lösungen in einer solchen Ausführungsform unterscheiden sich untereinander bevorzugt in ihrer Zusammensetzung, wobei die Salpetersäuregehalte der einzelnen Splitströme zwischen 0 und 30 Gew.% liegen können. Der zur Erstdispergierung verwendete Nitroniumionen-haltige Strom weist erfindungsgemäß jedoch mindestens einen Anteil von 20% an der dem Reaktor insgesamt zugeführten HNO₃ auf.

Der beim erfindungsgemäßen Verfahren eingesetzte Reaktor besteht aus aufeinander folgenden Reaktionsstufen, die den jeweils herrschenden Reaktionsgeschwindigkeiten und Stofftransporthemmungen angepaßt werden. Auf Grund der extrem stark abnehmenden Reaktionsgeschwindigkeit von der Mono- über die Dibis zur Trinitrierung müssen geeignete Maßnahmen zur optimalen Führung des Reaktionsprozesses getroffen werden. Durch die Wahl eines einzigen Reaktionsapparates wird die Mononitrierung bei sehr hohen Nitriergeschwindigkeiten durchgeführt. Zur Vermeidung einer unerwünscht hohen Nebenproduktausbeute ist es erfindungsgemäß notwendig, die beiden nichtmischbaren Phasen durch Dispergieren in sehr innigen Kontakt zu bringen und in weniger als einer Sekunde miteinander zu emulgieren. Zur Dispergierung von Aromaten in Mischsäure eignen sich alle Arten von Mischern. Solche Mischer sind aus Literaturangaben und Patenten bekannt [²³], [²⁴], [²⁵]. Bevorzugte Vorrichtungen zur schnellen Emulgierung der Eduktströme ineinander sind:
a) Strahlmischer
b) statische Mischer
c) dynamische Mischer.

Besonders bevorzugte Vorrichtungen zur schnellen Vermischung der Edukte und zum Starten der exothermen Nitrierreaktion stellen Strahlmischer dar, deren weitere Vorteile in der hermetischen Dichtheit und der variabel einstellbaren Mischleistung und der globalen Plug-Flow-Charakteristik liegen. Bevorzugt geeignete Geometrien von Strahlmischern sind beispielsweise aus den Patenten EP-A 0 101 007 (1982) und DE 37 44 001 bekannt.

In einer bevorzugten Ausführungsform werden die Edukte bei der Durchführung der ersten Nitrierung zur Verminderung unerwünschter Nebenreaktionen in einem schnell vermischenden Apparat des Typs Strahlmischer emulgiert und gleichzeitig und/oder anschließend wird die stark exotherme Erstnitrierung durchgeführt. Die dabei freiwerdende Wärme wird im Reaktionsgemisch gespeichert und bevorzugt nicht an die Umgebung abgegeben.

In einer besonders bevorzugten Ausführungsform wird die Mischungsintensität in dem Strahlmischer so gewählt, daß zunächst fast ausschließlich die Mononitrierung und anschließend die Dinitrierung im Reaktor abläuft, da die Temperatur der Reaktionsmischung während der Mononitrierung die Isomerenverteilung nach der Dinitrierung entscheidend beeinflußt. Erfindungsgemäß gelingt die Erstdispergierung der Reaktanden zu einer Emulsion als Reaktionsmedium in einem Strahlmischer mit einem spezifischen Energieeintrag von 10 bis 10000 Joule pro Liter, bevorzugt mit 50 bis 2 000 J/l, besonders bevorzugt mit 100 bis 1 000 J/l. Das wiederholte Redispergieren der koaleszierenden Emulsion durch Strahl-, statische und dynamische Mischer erfolgt bevorzugt bei einem spezifischen Energieeintrag von 1 bis 1 000 Joule pro Liter, besonders bevorzugt mit 10 bis 200 J/l.

Die Steuerung der Nitrierselektivität durch die Dispergierintensität, d.h. durch die unterschiedliche Stofftransporthemmung der chemischen Reaktionen, ist insbesondere bei dirigierenden Substituenten am Aromatenring von großer Bedeutung (beispielsweise bei Toluol und Chlorbenzol). In der bevorzugten Ausführungsform werden in weniger als einer Sekunde die nichtmischbaren Phasen ineinander emulgiert. Die durch den spezifischen Energieeintrag erzeugten feinen Emulsionstropfen in turbulenter Strömung sorgen somit für einen extrem schnellen Stoffaustausch zwischen organischer und wäßriger Phase, bewirken eine aufeinanderfolgende Mono- und Dinitrierung und vermeiden somit weitgehend eine gleichzeitige Mono- und Dinitrierung, die zu höheren mittleren Mononitriertemperaturen und damit bei vielen Aromaten zu verschlechterten Selektivitäten führt. Die Mononitrierung wird durch die schnelle Erstdispergierung und feine Emulgierung in weniger als 30 Sekunden, bevorzugt unter 15 Sekunden, beendet.

Zweiphasige Reaktionen mit einem gewünschten, praktisch vollständigen Umsatz bezüglich einer Komponente müssen zum Erzielen einer hohen Raum-Zeit-Ausbeute in einem die beiden Phasen vermischenden Reaktor mit einer Mindestverweilzeit durchgeführt werden. Solche Vorrichtungen sind in vielfältiger Weise bekannt [²⁶]. Geeignete Vorrichtungen zur simultanen Dispergierung mit garantierter Mindestverweilzeit sind
a) Verweilrohr mit dynamischen Mischern
b) Verweilrohr mit statischen Mischern
c) Verweilrohr mit Strahlmischern
d) Rührkesselkaskade
e) Blasensäule
f) Mehrstufiger Strahlmischer
oder eine Kombination der vorgenannten Reaktoren.

Für die Di- bzw. Mehrfachnitrierung erweist sich als bevorzugte Vorrichtung die Kombination eines Strahlmischers mit einem Rohrreaktor mit statischen Mischelementen. Durch das enge Verweilzeitspektrum wird der erforderliche Hold-Up des Reaktors minimal, so daß die Prozeßführung durch einen wenig trägen Reaktor deutlich erleichtert wird und die sicherheitstechnisch kritische Menge an heißer Nitriersäure klein gehalten werden kann. Außerdem ist eine solche Vorrichtung hermetisch dicht und kann kostengünstig aus emailliertem Stahl, Glas und/oder Tantal gefertigt werden.

Eine bevorzugte Vorrichtung zur Durchführung der Nitrierung stellt ein Rohrreaktor mit wiederkehrenden statischen Mischern oder Strahlmischern als Redispergatoren dar. Solche wiederkehrenden Verweilrohrabschnitte mit Redispergatoren werden hier als Reaktorstufen bezeichnet. In einer besonders bevorzugten Ausführungsform wird die Initialvermischung durch einen eine feine Emulsion erzeugenden Strahlmischer in einem Rohrreaktor mit 2 bis 30 Redispergierstufen durchgeführt. Bevorzugte Redispergatoren sind verschiedene Bauformen von Siebböden oder Lochblechen, welche die koaleszierende Emulsion bevorzugt bei einem spezifischen Energieeintrag von 1 bis 1000 Joule pro Liter, besonders bevorzugt mit 10 bis 200 J/l, wieder fein dispergiert.

Die Vermischungsleistung kann erfindungsgemäß entlang der Reaktorlängsachse mit zunehmendem Umsatz des Aromaten abnehmen, da die Reaktionsgeschwindigkeit zunehmend sinkt und die Stoffaustauschgeschwindigkeit durch die zunehmend bessere Löslichkeit der Aromaten, höhere Diffussionsgeschwindigkeit und gesteigerte Stabilität der Emulsion durch die zunehmend Grenzflächenstabilisierenden mehrfachnitrierten Aromatentröpfchen steigt. Deshalb kann es auch in einer ganz besonders bevorzugten Ausführungsform möglich sein, die Redispergierleistung im oberen Reaktorteil zu reduzieren, beispielsweise durch einen größeren Abstand der Redispergierstufen, durch weniger intensiv dispergierende Bauteile oder durch geringere Strömungsgeschwindigkeiten im Rohrreaktor und/oder im Redispergator. Die Verweilzeit zwischen den einzelnen Redispergierstufen wird erfindungsgemäß wesentlich von der Koaleszenzgeschwindigkeit der Emulsion bestimmt. Hohe Raum-Zeit-Ausbeuten lassen sich nur bei Vermeidung einer vollständigen Entmischung der Phasen vor erneuter Redispergierung erreichen. Die Verweilzeit zwischen den Dispergierstufen beträgt deshalb erfindungsgemäß zwischen 0,2 und 60 Sekunden. In bevorzugter Ausführungsform werden 0,5 bis 15 Sekunden als mittlere Verweilzeit zwischen den Dispergierstufen eingestellt.

Zur gezielteren Steuerung der Nitrierbedingungen der einzelnen Reaktionsschritte kann erfindungsgemäß die Nitroniumionen-haltige Lösung gesplittet und in verschiedener Konzentration dem Rohrreaktor zugeführt werden. Dabei kann in einer bevorzugten Ausführungsform die Zusammensetzung der Mischsäure variiert werden, um ein gegenüber der ungesplitteten Fahrweise günstigeren Temperaturund Salpetersäuregehalt-Verlauf entlang der Reaktorachse und damit eine verbesserte Selektivität der Nitrierung zu erreichen. In besonders bevorzugter Weise wird auch der Splitstrom mit einem Strahlmischer in den Reaktor eingetragen. Zur Erstdispergierung der Nitroniumion-haltigen Lösung mit Aromat werden mindestens 20% der gesamten HNO₃ verwendet.

Die Prozeßführung einer adiabatischen Dinitrierung ist gegenüber der konventionellen zweistufig-isothermen Nitrierung zu Dinitroaromaten weit überlegen, da die Temperatur- und damit energetische Steuerung ausschließlich durch die Zusammensetzung der Eduktströme und deren Temperatur geschieht. Zusammen mit den kurzen Verweilzeiten von bis zu 10 Minuten im Reaktor, bevorzugt von unter 5 Minuten, besonders bevorzugt von unter 3 Minuten, ergibt sich eine sehr leichte und schnelle Regelbarkeit über die Eduktströme eines solchen adiabatischen Mehrfachnitrierverfahrens.

Beim erfindungsgemäßen Verfahren werden als Nitroniumionen-haltige Lösungen Mischsäuren eingesetzt, die neben HNO₃ aus mindestens einer der folgenden Säuren bestehen: Essigsäure, Phosphorsäure, Perchlorsäure, Trifluormethansulfonsäure, Schwefelsäure. Die HNO₃ kann zur Bildung der Nitroniumhaltigen Lösung als freie Salpetersäure oder als eines ihrer Salze oder Ester oder anderer Verbindungen, die Salpetersäure entwickeln, eingesetzt werden. Bevorzugte Nitroniumionen-haltige Lösungen sind Mischsäuren von Salpetersäure, Essigsäure, Phosphorsäure und Schwefelsäure, wobei einzelne Säuren gegen höhere Bestandteile der verbleibenden ersetzt werden können. Erfindungsgemäß besonders bevorzugt besteht eine Nitroniumionen-haltige Lösung nur aus wäßriger Salpeter- und Schwefelsäure.

Die Mischsäure aus Salpeter- und Schwefelsäure enthält in einer besonders bevorzugten Ausführungsform, d.h. bei Vermeiden eines Splits der Nitroniumion-haltigen Lösung, 80 bis 100 Gew.% anorganische Bestandteile, die zu 60 bis 95 Gew.% aus H₂SO₄, 1 bis 20 Gew.% aus HNO₃ und mindestens 3 Gew.% aus H₂O bestehen. Sie besteht weiterhin aus bis zu 20 Gew.% organischen Bestandteilen, die sich zu 70 bis 100 Gew.% aus mehrfach nitriertem Aromaten und zum Rest aus Nebenprodukten zusammensetzen. Je nach gewünschtem Nitriergrad ist erfindungsgemäß das Molverhältnis zwischen Aromat und Salpetersäure in der Mischsäure zu wählen. In jedem Fall ist in dieser Lösung das Molverhältnis von HNO₃ zu Aromat von 1,3 bis 3,5, bevorzugt von 1,5 bis 3,0 und besonders bevorzugt von 1,7 bis 2,5 einzuhalten.

Strebt man eine vollständige Dinitrierung des Aromaten beispielsweise an, so wird theoretisch ein Molverhältnis HNO₃ : Aromat von 2,0 benötigt. Technisch geeignet sind Molverhältnisse von 1,3 bis 3,5, bevorzugt sind Molverhältnisse von 1,5 bis 3,0 und besonders bevorzugt sind Molverhältnisse von 1,7 bis 2,5 für die Dinitrierung anzuwenden. Stöchiometrie und Mischsäurezusammensetzung legen die Mengen der wäßrigen und organischen Phase und die adiabatische Temperaturerhöhung im Reaktor fest.

Die Reaktortemperatur wird durch die Mischtemperatur und die adiabatische Temperaturerhöhung der stark exothermen Reaktionen bestimmt. Im erfindungsgemäßen Verfahren liegt die Temperatur insgesamt zwischen 50 und 200°C. In einer bevorzugten Ausführungsform beträgt die Mischtemperatur der Reaktanden im Reaktorzulauf zwischen 80 und 150°C, bevorzugt zwischen 100 und 130°C, und die Temperatur der abfließenden Produkte zwischen 130 und 200°C, bevorzugt zwischen 140 und 180°C. In einer weiteren bevorzugten Ausführungsform, wobei gegenüber der ersten Variante deutlich mehr HNO₃ der Nitroniumionen-haltigen Lösung zugesetzt wird, beträgt die Mischtemperatur der Reaktanden im Reaktorzulauf zwischen 50 und 80°C und die Temperatur der abfließenden Produkte zwischen 130 und 200°C, bevorzugt zwischen 140 und 180°C.

Die kontinuierliche, adiabatische Dinitrierung gemäß diesem Verfahren kann mit allen Aromaten betrieben werden. Bevorzugte Aromaten jedoch sind Toluol, Benzol, Chlorbenzol und Xylol.

Durch die Kreisführung der Schwefelsäure nach der Aufarbeitung wird das sich in dieser lösende Produktgemisch nicht vollständig entfernt, so daß mit der Kreislaufschwefelsäure gelöste organische Produkte in den Reaktor zurückgeführt werden. Diese Produktkreisführung stört die erfindungsgemäße Reaktionsführung nicht.

Die Reaktionswärme der Erstnitrierung wird zusammen mit der intensiven Erstvermischung und wiederholten Redispergierung zur Herabsetzung der erforderlichen wasserbindenden Bestandteile in der Nitroniumionen-haltigen Lösung für die Zweitnitrierung genutzt In bevorzugter Ausführungsform kann der Schwefelsäure-Anteil in der Mischsäure gegenüber einem isothermen Verfahren um 1 bis 10 Gew.%-Punkte gesenkt werden. Weiterhin kann durch die adiabatische Verfahrensweise die Reaktionswärme vollständig zur Abtrennung des Produktwassers aus der Mischsäure genutzt werden, was eine mehrfache Nitrierung nach diesem Verfahren energetisch besonders günstig macht.

Im folgenden wird die Erfindung anhand von Ausführungsbeispielen und Zeichnungen näher erläutert.

Es zeigen
- Figur 1:: Ein Verfahrensschema für die adiabatische Dinitrierung in einem zweistufigen Düsenrohrreaktorsystem und
- Figur 2:: Ein Verfahrensschema für die adiabatische Dinitrierung in einem mehrstufigen Rohrreaktorsystem.

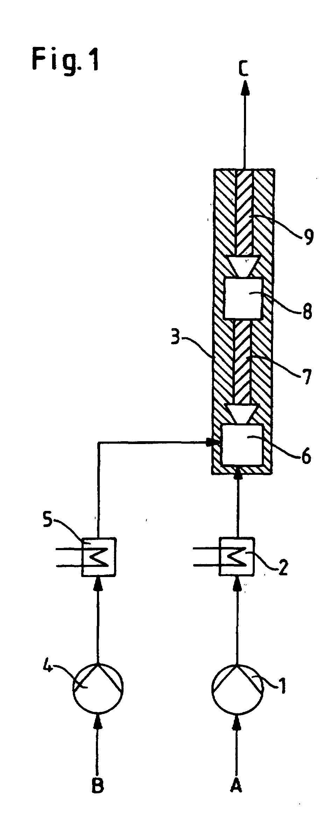

Gemäß Figur 1 wird ein aromatischer Flüssigkeitsstrom A mit einer Pumpe 1 durch einen Wärmetauscher 2 in das zweistufige Düsenrohrreaktorsystem 3 gedrückt. Im Wärmetauscher 2 wird das Edukt A auf die gewünschte Temperatur gebracht. Der Flüssigkeitsstrom B, bestehend aus der Nitroniumionen-haltigen Lösung, wird analog zu Strom A mit einer Pumpe 4 durch einen Wärmetauscher 5 in das zweistufige Düsenrohrreaktorsystem 3 gedrückt. Das zweistufige Düsenrohrreaktorsystem 3 besteht aus einer die beiden nichtmischbaren Ströme fein ineinander dispergierenden Düse 6, einem Verweilrohr 7, einer Redispergierdüse 8 und einem weiteren Verweilrohr 9. Das Reaktorsystem verläßt der Produktstrom C, bestehend aus einer wäßrigen und einer organischen Phase.

Die Düse 6 ist ein Strahlmischer, der am Düsenaustritt für eine sofortige intensive Vermischung beider Phasen sorgt, durch den hohen lokalen Energieeintrag die organische Phase zerteilt und in der wäßrigen Phase emulgiert. Gleichzeitig wird die exotherme Reaktion der Edukte A und B gestartet, die im Verweilrohr 7 abläuft. Die Dinitrierung verläuft in mehreren aufeinander folgenden Reaktionsschritten, wobei an den Aromatenring nacheinander Nitroniumionen addieren. Mit zunehmender Nitrogruppen-Zahl am Aromaten desaktiviert dieser, so daß die Nitriergeschwindigkeit zunehmend stark abfällt. Durch eine Koaleszenz der organischen Flüssigkeitströpfchen verkleinert sich die spezifische Oberfläche der Emulsion, so daß zur Aufrechterhaltung einer hohen Nitriergeschwindigkeit eine Redispergierung notwendig wird. In der auf das Verweilrohr 7 folgenden Redispergierdüse 8 werden die organischen Flüssigkeitströpfchen wieder zu einer feinen Emulsion in der wäßrigen Phase zerteilt. Die Nitrierreaktion wird in dem folgenden Verweilrohr 9 fortgeführt. Gegebenfalls können weitere Redispergier- und Verweilzeitstufen dahinter geschaltet werden, um einen vollständigen Umsatz des Aromaten zur dinitrierten Verbindung zu gewährleisten. Das erhaltene Produktgemisch aus wäßriger Restnitroniumionen-Lösung und aromatischer Phase wird anschließend in der für adiabatischen Nitrierreaktionen bekannten Weise aufgearbeitet (vgl. z.B. DE-A 42 38 390).

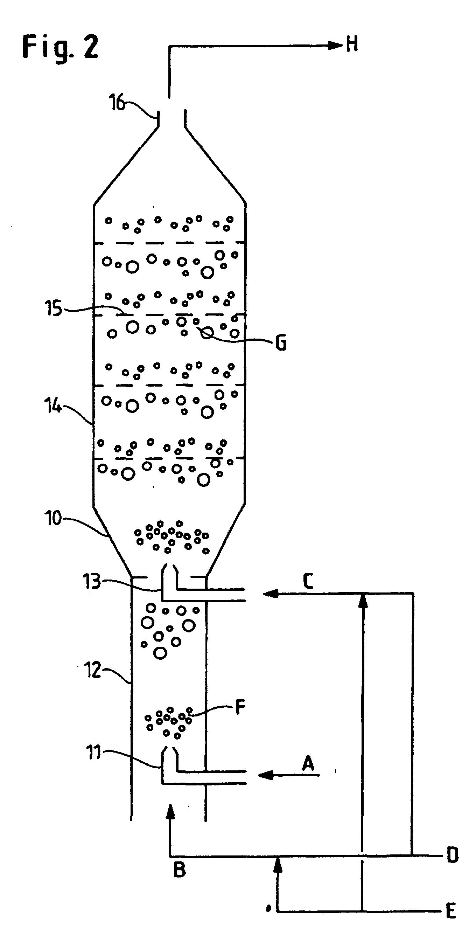

Gemäß Figur 2 kann die Dinitrierung eines Aromaten auch in einem Düsenrohrreaktor 10 mit mehrstufiger Redispergierung und initialer intensiver Dispergierung der beiden nichtmischbaren Flüssigkeitsströme durchgeführt werden. Die aromatische Verbindung A wird über den Strahlmischer 11 in die Nitroniumionen-haltige Lösung B geleitet. Die Nitroniumionen-haltigen Lösungen B und C können durch Mischen von wäßriger Salpetersäure D mit einer wäßrigen Hilfsstofflösung E hergestellt werden. Als wäßrige Salpetersäure D werden bevorzugt HNO₃-Gehalte zwischen 50 und 100 Gew.% eingesetzt. Die wäßrige Hilfsstofflösung E besteht bevorzugt neben Wasser aus H₂SO₄, kann aber auch zusätzlich oder vorwiegend H₃PO₄, CH₃COOH, Perchlorsäure und Trifluormethansulfonsäure enthalten. Der Flüssigkeitsstrom der aromatischen Verbindung A wird in dem Strahlmischer 11 des Düsenrohrreaktors 10 im Nitroniumionen-haltigen Strom B zerteilt und es entsteht eine feindisperse Emulsion F. Schon beim in Kontakt treten der beiden nichtmischbaren Flüssigkeiten A und B setzt die Reaktion zwischen Aromat und Nitroniumionen spontan ein. Die durch den Eintrag von kinetischer Energie geschaffene feine Emulsion F sorgt mit ihrer hohen Phasengrenzfläche für eine hohe Nitriergeschwindigkeit, die durch die Koaleszenz der organischen Flüssigkeitströpfen abnimmt.

Während die erste Nitrogruppe sehr schnell im kurzen Verweilrohr im unteren Reaktorteil 12 an den Aromaten addiert wird, benötigen die weiteren Nitrogruppen bei der Addition an den Aromaten eine längere Verweilzeit und schärfere Nitrierbedingungen. Zur Aufrechterhaltung einer hohen Umsetzungsgeschwindigkeit muß deshalb die koaleszierte Zweiphasenströmung G im Reaktionsraum wiederholt redispergiert werden. Die wesentlich schwierigere Di- und Trinitrierung wird durch die Exothermie der Nitrierreaktionen bei adiabatischer Betriebsweise des Düsenrohrreaktors begünstigt Zur Intensivierung der Nitrierbedingungen kann optional mittels eines zweiten Strahlmischers 13 frische Nitroniumionen-haltige Lösung C im Reaktor 10 verteilt werden. Gegebenenfalls kann dieses Zudosieren weiterer Nitroniumion-haltiger Lösung oder hochkonzentrierter Schwefelsäure analog zum Strom C im oberen Reaktorteil wiederholt werden. Die Zusammensetzung der Lösung C wird im allgemeinen nicht identisch mit der Lösung B sein und bevorzugt einen höheren HNO₃-Anteil besitzen. Im zweiten Reaktorteil 14 werden die koalezierten Aromatentröpfchen G wiederholt mit Redispergierorganen 15 (beispielsweise Lochbleche) in feindispersere Tröpfchen F zerteilt. Über den Reaktorauslaß 16 wird die Produktdispersion H erhalten. Die aus wäßriger Restnitroniumionen-Lösung und aromatischer Phase bestehende Produktdispersion wird anschließend in der für adiabatischen Nitrierreaktionen bekannten Weise aufgearbeitet (vgl. z.B. DE-A 42 38 390).

Die bei den Verfahrensschemen gemäß Figur 1 und 2 eingehaltenen Verfahrensparameter und Reaktionsbedingungen werden im folgenden anhand von Ausführungsbeispielen weiter erläutert.

### Beispiele:

Als Laborreaktor stand ein dreistufiger Düsenrohrreaktor gemäß der Figur 1 zur Verfügung. Die Eduktströme bestanden jeweils aus einem reinen Aromaten und einer Nitroniumionen-Lösung, die durch Mischung von wäßriger Salpetersäure mit wäßriger Schwefelsäure, sogenannter Mischsäure, hergestellt wurde. Die Eduktströme wurden gemäß Figur 1 dosiert und thermostatisiert. Der Düsenrohrreaktor wurde isoliert, um einen Temperaturverlust bei adiabatischem Betrieb zu vermeiden. Die Zusammensetzung der Mischsäure wurde gravimetrisch hergestellt. Nach Trennung der wäßrigen und organischen Phase wurde die organische Phase zunächst mit 10%iger wäßriger Sodalösung und anschließend zweimal mit Wasser gewaschen. Die Analyse der aufgearbeiteten organische Phase erfolgte gaschromatographisch mittels einer Glaskapillarsäule, wobei die Komponenten in Flächenprozent bestimmt wurden. Durch die adiabatische Betriebsweise werden die Reaktionstemperaturen entlang des Reaktorsystems ausschließlich durch die Zusammensetzung der Stoffströme, den Umsatzgrad und die Temperatur der Edukte bestimmt. Als Reaktorstufe wird jeweils eine Dispergierdüse mit nachgeschaltetem Verweilrohr bezeichnet.

### Beispiel 1:

Es wurde ein dreistufiger Düsenrohrreaktor mit einer ersten Mischdüse mit einem Innendurchmesser von 0,3 mm und zwei Redispergierdüsen mit einem Innendurchmesser von 0,3 mm verwendet. Hinter jeder Düse wurde ein 60 mm langes Verweilrohr mit einem Innendurchmesser von 4 mm installiert. Die Edukttemperatur betrugen jeweils 120°C vor der Vermischung und die beiden Stoffströme wurden wie folgt gewählt: 112,4 ml/h (97,4 g/h = 1,06 mol/h) Toluol und 2500 ml/h (4330 g/h = 2,27 mol/h) einer Nitriersäure der Zusammensetzung 78,3:3,3:18,4 (Gew.% H₂SO₄:HNO₃:H₂O).

Es wurden die folgenden Produktverteilungen nach der ersten, zweiten und dritten Reaktorstufe (jeweils nach Düse und Verweilzeitstrecke) gaschromatographisch gemessen (MNT = Mononitrotoluol, DNT = Dinitrotoluol, TNT = Trinitrotoluol, Rest: Nebenkomponenten):

| | 1. STUFE | 2. STUFE | 3. STUFE |
|---|---|---|---|
| 2-MNT | 6,6% | 0,3% | - |
| 3-MNT | 0,9% | 0,1% | - |
| 4-MNT | 4,7% | 0,5% | - |
| 2,6-DNT | 16,7% | 18,8% | 19,0% |
| 2,5-DNT | 0,9% | 1,1% | 1,1% |
| 2,4-DNT | 65,0% | 73,6% | 74,0% |
| 2,3-DNT | 1,9% | 2,2% | 2,3% |
| 3,4-DNT | 2,8% | 3,3% | 3,4% |
| 2,4,6-TNT | - | - | - |

### Beispiel 2:

Es wurde der Reaktor aus Beispiel 1 verwendet Die Edukttemperatur betrug in diesem Fall jeweils 70°C und die beiden Stoffströme wurden wiefolgt gewählt: 315,7 ml/h (273,7 g/h = 2,97 mol/h) Toluol und 4000 ml/h (6924 g/h = 6,37 mol/h) einer Nitriersäure der Zusammensetzung 76,9:5,8:17,3 (Gew.% H₂SO₄:HNO₃:H₂O).

Es wurden die folgenden Produktverteilungen nach der ersten, zweiten und dritten Reaktorstufe (jeweils nach Düse und Verweilzeitstrecke) gaschromatographisch gemessen (MNT = Mononitrotoluol, DNT = Dinitrotoluol, Rest: Nebenkomponenten):

| REAKTORSTUFE | SUMME MNT | SUMME DNT |
|---|---|---|
| 1 | 26,5% | 73,4% |
| 2 | 16,7% | 83,2% |
| 3 | 9,1% | 90,8% |

### Beispiel 3:

Es wurde der dreistufige Düsenrohrreaktor aus Beispiel 1 modifiziert mit einer ersten Mischdüse mit einem Innendurchmesser von 0,2 mm und einer Redispergierdüse mit einem Innendurchmesser von 0,2 mm verwendet. Hinter der ersten Düse wurde ein 605 mm langes Verweilrohr und hinter der zweiten Düse ein Verweilzeitrohr der Länge 605 mm mit jeweils einem Innendurchmesser von 2 mm und dahinter ein weiteres 2300 mm langes Verweilrohr mit einem Innendurchmesser von 1,76 mm installiert. Die Edukttemperatur betrug jeweils 120°C und die Stoffströme wurden wiefolgt gewählt: 301,8 ml/h (261,6 g/h = 2,84 mol/h) Toluol und 5500 ml/h (9423,7 g/h = 5,38 mol/h) einer Nitriersäure der Zusammensetzung 77,0:3,6:19,4 (Gew.% H₂SO₄:HNO₃:H₂O).

Es wurden die folgende Produktverteilung nach der dritten Reaktorstufe gaschromatographisch gemessen (MNT = Mononitrotoluol, DNT = Dinitrotoluol, Rest: Nebenkomponenten):

| Reaktorstufe | Summe MNT | Summe DNT |
|---|---|---|
| 3 | 13,2% | 86,5% |

### Beispiel 4:

Es wurde der dreistufige Düsenrohrreaktor aus Beispiel 1 modifiziert mit einer ersten Mischdüse mit einem Innendurchmesser von 0,2 mm und einem nachgeschalteten Verweilrohr von 550 mm Länge bei 2 mm Innendurchmesser sowie zwei Redispergierdüsen mit einem Innendurchmesser von 0,3 mm und einem jeweils nachgeschalteten Verweilrohr von 220 mm Länge bei 2 mm Innendurchmesser verwendet Die Edukttemperatur betrug jeweils 120°C und die Stoffströme wurden wiefolgt gewählt: 121,3 mol/h (104,88 g/h = 1,14 mol/h) Toluol und 2500 mol/h (4283,5 g/h = 2,45 mol/h) einer Nitriersäure der Zusammensetzung 77,0:3,6:19,4 (Gew.% H₂SO₄:HNO₃:H₂O).

Es wurden die folgenden Produktverteilungen nach der ersten, zweiten und dritten Reaktorstufe (jeweils nach Düse und Verweilzeitstrecke) gaschromatographisch gemessen (MNT = Mononitrotoluol, DNT = Dinitrotoluol, Rest: Nebenkomponenten):

| Reaktorstufe | Summe MNT | Summe DNT |
|---|---|---|
| 1 | 29,9% | 69,9% |
| 2 | 8,6% | 91,3% |
| 3 | 4,7% | 95,2% |

### Beispiel 5:

Es wurde der Reaktor aus Beispiel 4 verwendet. Die Edukttemperatur betrug jeweils 120°C und die Stoffströme wurden wie folgt gewählt: 167,8 g/h (= 1,82 mol/h) Toluol und 6853,6 g/h (= 3,92 mol/h) einer Nitriersäure der Zusammensetzung 77,0:3,6:19,4 (Gew.% H₂SO₄:HNO₃:H₂O).

Es wurden die folgenden Produktverteilungen nach der ersten, zweiten und dritten Reaktorstufe (jeweils nach Düse und Verweilzeitstrecke) gaschromatographisch gemessen (MNT = Mononitrotoluol, DNT = Dinitrotoluol, Rest: Nebenkomponenten):

| Reaktorstufe | Summe MNT | Summe DNT |
|---|---|---|
| 1 | 28,1% | 71,6% |
| 2 | 9,0% | 90,8% |
| 3 | 5,1% | 94,8% |

### Beispiel 6:

Es wurde der Reaktor aus Beispiel 4 verwendet. Die Edukttemperatur betrug jeweils 120°C und die Stoffströme wurden wie folgt gewählt: 230,7 g/h (= 2,51 mol/h) Toluol und 9423,7 g/h (= 5,38 mol/h) einer Nitriersäure der Zusammensetzung 77,0:3,6:19,4 (Gew.% H₂SO₄:HNO₃:H₂O).

Es wurden die folgenden Produktverteilungen nach der ersten, zweiten und dritten Reaktorstufe (jeweils nach Düse und Verweilzeitstrecke) gaschromatographisch gemessen (MNT = Mononitrotoluol, DNT = Dinitrotoluol, Rest: Nebenkomponenten):

| REAKTORSTUFE | SUMME MNT | SUMME DNT |
|---|---|---|
| 1 | 27,5% | 72,4% |
| 2 | 10,3% | 88,9% |

### Beispiel 7:

Es wurde der Reaktor aus Beispiel 4 modifiziert, in dem zusätzlich hinter die Misch- und die beiden Redispergierdüsen weitere drei Redispergierdüsen mit einem Innendurchmesser von 0,3 mm sowie ein jeweils anschließendes 550 mm langes Verweilrohr mit einem Innendurchmesser von 2 mm verwendet wurde. Die Edukttemperatur betrug jeweils 140°C und die Stoffströme wurden wiefolgt gewählt: 78,0 g/h (=1,0 mol/h) Benzol und 2500 g/h (= 2,3 mol/h) einer Nitriersäure der Zusammensetzung 78,6:5,8:15,6 (Gew.% H₂SO₄:HNO₃:H₂O).

Es wurden die folgende Produktverteilung nach der sechsten Reaktorstufe gaschromatographisch gemessen (MNB = Mononitrobenzol, DNB = Dinitrobenzol):

| Reaktorstufe | Summe MNB | Summe DNB |
|---|---|---|
| 6 | 1,3% | 98,7% |

### Beispiel 8:

Es wurde der Reaktor aus Beispiel 7 verwendet. Die Edukttemperatur betrug jeweils 135°C und die Stoffströme wurden wiefolgt gewählt: 112,5 g/h (=1,0 mol/h) Chlorbenzol und 2500 g/h (= 2,3 mol/h bezogen auf HNO₃) einer Nitriersäure der Zusammensetzung 81,5:5,8:12,7 (Gew.% H₂SO₄:HNO₃:H₂O).

Es wurden die folgende Produktverteilung nach der sechsten Reaktorstufe gaschromatographisch gemessen (MNCB = Mononitrochlorbenzol, DNCB = Dinitrochlorbenzol, Rest: Nebenprodukte):

| Reaktorstufe | Summe MNCB | Summe DNCB |
|---|---|---|
| 6 | 78,2% | 21,6% |

[1] Muspratt & Hofmann: Liebigs Ann. Chem. 57 (1846), 201
[2] Kirk-Othmer; Encyclopedia of Chemical Technology, 3rd. ed. (1981), Vol. 15
[3] Ullmann; Encyclopedia of Industrial Chemistry, Vol. A17, 411-455, VCH Weinheim (1991)
[4] Groggins: Unit Processes in Organic Chemistry, McGraw-Hill, New York (1958)
[5] Hansen et al.: Chem. Eng. Sci., 32 (1977), 775
[6] Albright et al.: ACS Symposium Series 22, American Chemical Society (1976), 201
[7] Albright et al.: J. App. Chem. Biotechnol., 26 (1976), 522
[8] Urbanski: Chemistry and Technology of Explosives, Vol. 1, MacMillan, New York (1964)
[9] Albright et al.: ACS Symposium Series 22, American Chemical Society (1976), 201
[10] Urbanski: Chemistry and Technology of Explosives, Vol. 1, MacMillan, New York (1964)
[11] Ullmann; Encyclopedia of Industrial Chemistry, Vol. A17, 411-455, VCH Weinheim (1991)
[12] Chem.-Ing.-Techn. 56 (1984), 552-553
[13] Ullmann; Encyclopedia of Industrial Chemistry, Vol. B4, 561-586, VCH Weinheim (1992)
[14] Koglin et al.; Chem.-Ing.-Techn. 53 (1981), 641-647
[15] Ullmann; Encyclopedia of Industrial Chemistry, Vol. B2, Chap. 24, VCH Weinheim (1992)
[16] Bourne & Maire: Chem. Eng. Process 30 (1991) 23
[17] Brodkey: Chem. Eng. Commun. 8 (1981) 1
[18] Brodkey (ed.): Turbulence in Mixing Operations - Theory and Application to Mixing and Reaction, Academic Press, New York (1975)
[19] Ullmann; Encyclopedia of Industrial Chemistry, Vol. B4, 561-586 VCH Weinheim (1992)
[20] Schröder et al.; Chem.-Ing.-Techn. 56 (1984) 552-553
[21] Koglin et al.; Chem.-Ing.-Techn. 53 (1981) 641-647
[22] Koglin; Maschinenmarkt 86 (1980) 346-350
[23] Ullmann; Encyclopedia of Industrial Chemistry, Vol. B4, 561-586, VCH Weinheim (1992)
[24] Ullmann; Encyclopedia of Industrial Chemistry, Vol. B2, Chap. 24, VCH Weinheim (1992)
[25] Perry's Chemical Engineers' Handbook, 6ed. (1984), McGraw-Hill, New York 21-61
[26] Ullmann; Encyclopedia of Industrial Chemistry, Vol. B4, 87-120, VCH Weinheim (1992)

## Patentansprüche

1. Kontinuierliches Verfahren zur Dinitrierung von aromatischen Verbindungen durch Reaktion mit einer Nitroniumionen-haltigen Lösung, **dadurch gekennzeichnet, daß**
a) die Dinitrierung in einem einzigen Apparat unter adiabatischen Bedingungen in einer Emulsion als Reaktionsmedium durchgeführt wird,
b) in den Reaktor pro Mol aromatische Verbindung 1,3 bis 3,5 Mol HNO₃ in Form einer Nitroniumionen-haltigen Lösung dosiert werden,
c) die zur Koaleszenz neigende Emulsion durch mehrfaches Dispergieren aufrechterhalten wird,
d) das erstmalige Dispergieren der Flüssigkeitsströme in weniger als einer Sekunde zur Herstellung der Emulsion durchgeführt wird,
e) zum erstmaligen Dispergieren mindestens 20% der gesamten HNO₃ eingesetzt werden kann.

2. Verfahren gemäß Anspruch 1), **dadurch gekennzeichnet, daß** die Nitroniumionen-haltige Lösung vollständig zur erstmaligen Dispergierung der beiden Phasen eingesetzt wird.

3. Verfahren gemäß Anspruch 1) oder 2), **dadurch gekennzeichnet, daß** die Nitroniumionen-haltige Lösung eine Mischsäure aus wäßriger Schwefel- und Salpetersäure ist.

4. Verfahren gemäß einem der Ansprüche 1) bis 3), **dadurch gekennzeichnet, daß** pro Mol aromatische Verbindung 1,5 bis 3,0 Mol HNO₃, bevorzugt 1,7 bis 2,5 Mol HNO₃, in der Nitroniumionen-haltigen Lösung vorliegen.

5. Verfahren gemäß einem der Ansprüche 1) bis 4), **dadurch gekennzeichnet, daß** die Erstdispergierung der Reaktanden in einem Strahlmischer mit einem spezifischen Energieeintrag von 10 bis 10 000 Joule pro Liter, bevorzugt mit 50 bis 2 000 J/l, besonders bevorzugt mit 100 bis 1 000J/l zu einer Emulsion führt.

6. Verfahren gemäß einem der Ansprüche 1) bis 5), **dadurch gekennzeichnet, daß** die wiederholte Redispergierung der Emulsion durch Strahl-, statische oder dynamische Mischer erfolgt, bevorzugt bei einem spezifischen Energieeintrag von 1 bis 1 000 Joule pro Liter, besonders bevorzugt mit 10 bis 200 J/l.

7. Verfahren gemäß Anspruch 2), **dadurch gekennzeichnet, daß** die Nitroniumionen-haltige Lösung aus 80 bis 100 Gew.% anorganischen Anteilen besteht, die sich aus 60 bis 95 Gew.% Schwefelsäure, I bis 20 Gew.% Salpetersäure und mindestens 3 Gew.% Wasser zusammensetzen, sowie aus bis zu 20 Gew.% organischen Bestandteilen, die sich zu 70 bis 100 Gew.% aus dinitrierten Aromaten und zum Rest aus Nebenprodukten zusammensetzen.

8. Verfahren gemäß einem der Ansprüche 1) bis 7), **dadurch gekennzeichnet, daß** die Mischtemperatur der Reaktanden zwischen 80 und 150°C, bevorzugt zwischen 100 und 130°C, liegt und daß die Reaktanden bei Temperaturen von 130 bis 200°C, bevorzugt bei 140 bis 180°C, den Reaktor verlassen.

9. Verfahren gemäß einem der Ansprüche 1) bis 7), **dadurch gekennzeichnet, daß** die Mischtemperatur der Reaktanden zwischen 50 und 80°C liegt und daß die Reaktanden bei Temperaturen von 130 bis 200°C, bevorzugt bei 140 bis 180°C, den Reaktor verlassen.

10. Verfahren gemäß einem der Ansprüche 1) bis 9), **dadurch gekennzeichnet, daß** es sich bei dem Aromaten bevorzugt um Toluol, Benzol, Chlorbenzol oder Xylol handelt.

## Claims

1. Continuous process for the dinitration of aromatic compounds by reaction with a nitronium ion solution, **characterised in that**
a) the dinitration is carried out in a single apparatus under adiabatic conditions in an emulsion as the reaction medium,
b) from 1.3 to 3.5 mol of HNO₃ per mol of aromatic compound is fed in the form of a nitronium ion solution to the reactor,
c) the emulsion, which has a tendency to coalescence, is maintained by repeated dispersion,
d) the first dispersion of the liquid streams to produce the emulsion is carried out in less than one second,
e) at least 20% of the entire HNO₃ can be used for the first dispersion.

2. Process according to claim 1, **characterised in that** the nitronium ion solution is used entirely for the first dispersion of the two phases.

3. Process according to claim 1 or 2, **characterised in that** the nitronium ion solution is a mixed acid composed of sulfuric acid and nitric acid.

4. Process according to any one of claims 1 to 3, **characterised in that** from 1.5 to 3.0 mol of HNO₃, preferably from 1.7 to 2.5 mol of HNO₃, per mol of aromatic compound is present in the nitronium ion solution.

5. Process according to any one of claims 1 to 4, **characterised in that** the first dispersion of the reactants in a jet mixer with a specific energy input of from 10 to 10,000 joules per litre, preferably of from 50 to 2,000 J/1, particularly preferably of from 100 to 1,000 J/1, results in an emulsion.

6. Process according to any one of claims 1 to 5, **characterised in that** the repeated redispersion of the emulsion is carried out by means of jet mixers, static mixers or dynamic mixers, preferably at a specific energy input of from 1 to 1,000 joules per litre, particularly preferably of from 10 to 200 J/l.

7. Process according to claim 2, **characterised in that** the nitronium ion solution consists of from 80 to 100 wt.% of inorganic constituents, which are composed of from 60 to 95 wt.% of H₂SO₄, 1 to 20 wt.% of HNO₃ and at least 3 wt.% of H₂O and also of up to 20 wt.% of organic constituents, which are composed of from 70 to 100 wt.% of polynitrated aromatics with the remainder being by-products.

8. Process according to any one of claims 1 to 7, **characterised in that** the mixing temperature of the reactants is between 80 and 150°C, preferably between 100 and 130°C, and that the reactants leave the reactor at temperatures of from 130 to 200°C, preferably at 140 to 180°C.

9. Process according to any one of claims 1 to 7, **characterised in that** the mixing temperature of the reactants is between 50 and 80°C and that the reactants leave the reactor at temperatures of from 130 to 200°C, preferably at 140 to 180°C.

10. Process according to any one of claims 1 to 9, **characterised in that** the aromatics are preferably toluene, benzene, chlorobenzene or xylene.

## Revendications

1. Procédé continu pour la dinitration de composés aromatiques par la réaction avec une solution contenant des ions nitronium, **caractérisé en ce que** :
a) la dinitration est réalisée dans un seul appareil dans des conditions adiabatiques, dans une émulsion comme milieu de réaction ;
b) dans le réacteur, on ajoute par mole de composé aromatique, 1,3 à 3,5 moles de HNO₃ sous la forme d'une solution contenant des ions nitronium ;
c) l'émulsion tendant à la coalescence est conservée par plusieurs dispersions ;
d) la première dispersion du courant liquide est réalisée en moins d'une seconde pour la préparation de l'émulsion, et
e) pour la première dispersion, on peut mettre en oeuvre au moins 20% du HNO₃ total.

2. Procédé suivant la revendication 1, **caractérisé en ce que** la solution contenant des ions nitronium est mise en oeuvre complètement pour la première dispersion des deux phases.

3. Procédé suivant la revendication 1 ou 2, **caractérisé en ce que** la solution contenant des ions nitronium est un acide mixte d'acides sulfurique et nitrique aqueux.

4. Procédé suivant l'une quelconque des revendications 1 à 3, **caractérisé en ce que** par mole de composé aromatique, sont présentes 1,5 à 3,0 moles de HNO₃, de préférence 1,7 à 2,5 moles de HNO₃, dans la solution contenant des ions nitronium.

5. Procédé suivant l'une quelconque des revendications 1 à 4, **caractérisé en ce que** la première dispersion des réactifs dans un mélangeur à jet avec un apport spécifique d'énergie allant de 10 à 10 000 joules par litre, de préférence de 50 à 2000 J/litre, de manière particulièrement préférée de 100 à 1000 J/litre, conduit à une émulsion.

6. Procédé suivant l'une quelconque des revendications 1 à 5, **caractérisé en ce que** la redispersion de l'émulsion est réalisée par un mélangeur à jet, statique ou dynamique, de préférence avec un apport spécifique d'énergie de 1 à 1000 joules par litre, de manière particulièrement préférée de 10 à 200 J/litre.

7. Procédé suivant la revendication 2, **caractérisé en ce que** la solution contenant des ions nitronium consiste en 80 à 100% de fraction inorganique, qui se composent de 60 à 95% en poids d'acide sulfurique, 1 à 20% en poids d'acide nitrique et au moins 3% en poids d'eau, ainsi que de jusqu'à 20% en poids de constituants organiques, qui se composent de 70 à 100% en poids d'aromatiques dinitrés et pour le reste, de produits secondaires.

8. Procédé suivant l'une quelconque des revendications 1 à 7, **caractérisé en ce que** la température de mélange des réactifs se situe dans l'intervalle allant de 80 à 150°C, de préférence de 100°C à 130°C et les réactifs quittent le réacteur à des températures allant de 130 à 200°C, de préférence de 140 à 180°C.

9. Procédé suivant l'une quelconque des revendications 1 à 7, **caractérisé en ce que** la température de mélange des réactifs se situe dans l'intervalle allant de 50 à 80°C et que les réactifs quittent le réacteur à des températures allant de 130 à 200°C, de préférence de 140 à 180°C.

10. Procédé suivant l'une quelconque des revendications 1 à 9, **caractérisé en ce que** les aromatiques consistent de préférence en le toluène, le benzène, le chlorobenzène ou le xylène.
